# EUROPEAN PATENT APPLICATION

(11) **EP 4 047 100 A1**
(43) Date of publication of application: **24.08.2022**
(21) Application number: 21157747.3
(22) Date of filing: 18.02.2021
(51) Int. Cl.: C12Q 1/6844, C12Q 1/6874

(54) **NGS TARGETED DNA PANEL USING MULTIPLEX SELECTIVE RCA**

(71) Applicant: Miltenyi Biotec B.V. & Co. KG, 51429 Bergisch Gladbach (DE)
(72) Inventor: The designation of the inventor has not yet been filed
(74) Representative: Kisters, Michael Marcus

(57) **Abstract**

Next generation sequencing (NGS) is a powerful tool to study clinically relevant mutations and targeted DNA panels which are commonly used requires the specific amplification of DNA fragments (amplicons). The two most commonly used techniques for NGS target enrichment are multiplex polymerase chain reaction (PCR-based amplicon sequencing) and hybrid capture-based enrichment (HCBE). In the present invention we describe a method using rolling circle amplification to selectively amplify double stranded DNA nucleic acid library and sequence a DNA panel composed of target specific fragments.

SEQUENCE LISTING

<110> Miltenyi Biotec B.V. & Co. KG

<120> NGS targeted DNA panel using multiplex selective RCA

<130> MBG_62

<160> 8

<170> BiSSAP 1.3.6

<210> 1
<211> 22
<212> DNA
<213> Homo sapiens


<400> 1
attgagtacc caccactagc ca                                                22


<210> 2
<211> 28
<212> DNA
<213> Homo sapiens


<400> 2
tggctaattg agtacccacc actagcca                                          28


<210> 3
<211> 20
<212> DNA
<213> Homo sapiens


<400> 3
ggtccctcca gctactgctc                                                   20


<210> 4
<211> 23
<212> DNA
<213> Homo sapiens


<400> 4
gagggtccct ccagctactg ctc                                               23


<210> 5
<211> 34
<212> DNA
<213> Homo sapiens


<400> 5
tgctgagtga aagaggatat atttattggc tgag                                    34


<210> 6

<211> 40
<212> DNA
<213> Homo sapiens

<400> 6
ctcagctgct gagtgaaaga ggatatattt attggctgag 40

<210> 7
<211> 22
<212> DNA
<213> Homo sapiens

<400> 7
ggcctaggga actccatctt gt 22

<210> 8
<211> 28
<212> DNA
<213> Homo sapiens

<400> 8
acaagaggcc tagggaactc catcttgt 28

## Description

### BACKGROUND

Next generation sequencing (NGS) is an extremely powerful tool to study clinically relevant target sequences and gene-base panel tests are the first choice for studying DNA variants. These panels require the specific/targeted amplification of DNA fragments (amplicons). The two most commonly used techniques for NGS target enrichment are multiplex polymerase chain reaction (PCR-based amplicon sequencing) and hybrid capture-based enrichment (HCBE).

Multiplex PCR are often used for target region of less 1Mb target region for the detection of single nucleotide polymorphisms (SNPs) and small insertions/deletions. On the other hand, the hybrid capture method is used more often for large target region and for the detection of fusion genes, copy number variations (CNV) for instance.

While PCR and HCBE have been highly useful and effective at creating DNA copies or segregating specific portions of DNA for sequencing purposes, both required the generation of adapted DNA libraries and the design of specific DNA primers and/or specific capture oligos.

In other words, there is a need for a simple method to specifically amplify and optionally detect/sequence target DNA out of a sample comprising non-target DNA. With such method, target DNA could be multiplied as often as necessary to obtain an accurate and reliable amount over non-target DNA.

### SUMMARY

The present invention is directed to a process for amplification of a panel of DNA moieties that are generated specifically and selectively from a simple shotgun DNA library using Rolling circle amplification (RCA) and target specific sequencing.

Object of the invention is a method for selective amplifying target double-stranded DNA molecules from a sample comprising target and non-target DNA molecules **characterized by** subsequently
a. providing target and non-target DNA molecules at the 3' and 5' ends with barcoding regions A1 and A2 each comprising 5 to 50 nucleotides thereby obtaining barcoded DNA molecules
b. providing barcoded DNA molecules with oligonucleotide guides comprising 5 to 50 nucleotides capable of binding to the regions A1 and A2 of the same barcoded DNA molecule and circularizing the barcoded DNA molecules by ligation with a DNA ligase using the guide oligonucleotides thereby obtaining circular templates
c. providing the guide oligonucleotides of the circular templates with a blocking molecule
d. providing the circular templates with a primer oligonucleotide comprising 5 to 50 nucleotides capable of binding at least a part of selected target DNA molecules thereby obtaining primed circular target DNA molecules.
e. multiplying the primed circular target DNA molecules into DNA concatemers by (rolling circle amplification) RCA reaction.

Rolling circle amplification (RCA) is a method that is used routinely to generate hundreds of replicate of the same circularized molecule with high accuracy.

Briefly, a DNA sample is fragmented randomly to about 200 nucleotides long DNA pieces and tagged to generate adapter-tagged libraries. The adapted library is circularized and the DNA is then used as a template for RCA. The RCA reaction needs to be primed using an oligonucleotide (RCA primers) that is complementary to a portion of the circularized template DNA. This short duplex/circular template is recognized by the Polymerase performing the RCA. In the current invention, a series of specific primers designed to target portion of the fragments corresponding to the genes of interest is used to prime a multiplex RCA reaction instead of using a universal primer that would recognize the common adapter portion of the circularized DNA library and amplifying the DNA regardless of the target sequence.

The specific RCA primers would be designed as a target-specific panel that would be the equivalent to using a series of primers in a highly multiplex PCR reaction to amplify selectively and specifically a subset of the genome but applied to an RCA reaction.

With the current approach, the RCA reaction will produce long repeating product strands that serve as amplified copies of the circle sequence. These DNA concatemers of the same molecule will form DNA nanoballs or rolonies corresponding to only a subset of all fragmented and circularized DNA.

### BRIEF DESCRIPTION OF THE DRAWINGS

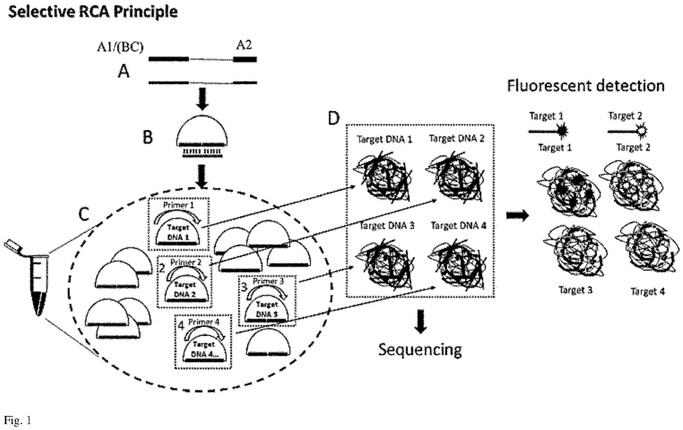

Fig. 1 shows the process of the invention where DNA libraries (A) are used to generate sense and anti-sense circular templates (B). Target specific oligonucleotides hybridized to a subset of circles corresponding to the target DNA of interest and the duplexes formed on each circle served as starting point for the strand-displacement polymerase that is used in the rolling circle amplification (RCA) approach (C) . The RCA generates concatemers forming DNA nanoballs called rolonies (D). The generated rolonies correspond to only the target DNA of interest and can be subsequently sequenced or detected via hybridization using fluorescently labeled oligonucleotide complementary to the amplified DNA fragment of interest.

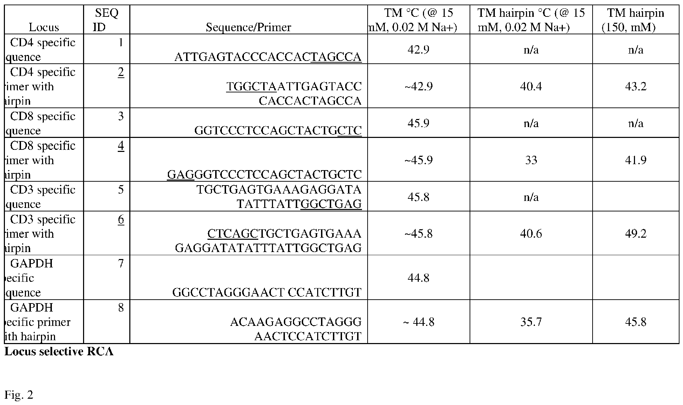

Fig 2. show an example of a set of primers used for selectively amplify circularized templates. The primer oligonucleotides hybridize to a defined portion of CD3, CD4, CD8 and GPDH genes under conditions allowing for the specific annealing to the single stranded circle are depicted (locus selective RCA).

### DETAILED DESCRIPTION

### Step a)

In step a), the target and non-target DNA molecules are providing at the respective 3' and or 5' ends with adaptor DNA molecules containing barcoding regions and common sequences A1 and A2, thereby obtaining barcoded DNA molecules. A1 and A2 may each comprise 5 to 100 nucleotides. The barcode adapters are added enzymatically by ligation of short double-stranded oligonucleotides using a DNA ligase.

In a variant of the invention, A1 comprises further a random DNA sequence region of 5 to 100 nucleotides.

After step a) the sample comprises at least in part molecule with the general formula A1- target DNA- A2 and A1-non-target DNA-A2. The orientation of A1/A2 to the respective 3' and 5' ends of the DNA molecule is not important.

In a first embodiment of the method, the sample comprising target and non-target oligonucleotides can be obtained by segmentation/fragmentation of a double stranded DNA nucleic acid library.

In a second embodiment of the method the sample comprising target and non-target non-target DNA molecules is obtained by denaturation of a double stranded DNA nucleic acid library into a mixture of sense and anti-sense DNA single strands comprising target and non-target non-target DNA molecules.

### Step b

In step b), the barcoded DNA molecules are provided with oligonucleotide guides capable of binding to the barcoding regions A1 and A2 of the same barcoded DNA molecule. This procedure "bends" the common portion of the barcoded DNA molecules into a circle or padlock which is then circularized by ligation using the guide oligonucleotides with a DNA ligase into circular templates. The oligonucleotide guide comprises at least 5 and preferably up to 50 nucleotides. The circularization may be performed with a DNA ligase like a T4 DNA ligase and can be followed by a clean-up step involving treatment with exonuclease I, III, as well as Lambda exonuclease to eliminate superfluous DNA, that may be phosphorylated or non-phosphorylated and non-circular DNA, as well as excess bridge oligonucleotides.

The DNA ligase recognizes the DNA duplex form the two extremities brought in close proximity to one another by the hybridization via the guide DNA oligonucleotide. The ligation will ligate the two extremities which will then form a circularized and closed DNA molecule.

The guide oligonucleotide is usually provided in vast excess and is preferentially 5' phosphorylated and is a good substrate for Lambda Exonuclease during circle clean-up. Separation of ssDNA and enzymes can be accomplished by several methods known to experts in the field, for example Ampure beads (Agencourt) or spin columns with a silicon matrix (e.g. QIAquick PCR purification Columns, QIAGEN)

### Step c

To avoid non-specific RCA priming via still-attached guide oligos, the guide oligo is blocked to prevent its usage as a potential primer of subsequent RCA reactions like in step e). To this end, in step c, the guide oligonucleotides of the circular templates may be provided at the 3'end with a blocking molecule wherein the blocking molecule is a dideoxynucleotides capable of blocking the guide oligonucleotides as primer for the RCA reaction in step e).

### Step d

In step d), the circular templates in a low salt solution are hybridized with a primer oligonucleotide comprising 5 to 50 nucleotides capable of binding at least a part of the target DNA molecules obtaining primed circular target DNA molecules. The primer oligonucleotide serve as sequencing primers for the subsequent RCA multiplication step.

RCA primer sequences are selected to provide good specificity at the intended hybridization temperatures. They are then modified to form hairpins at the temperature and salt concentration of rolonization in the absence of specific binding. The modification may involve addition of a 3' inverse complementary region to the 5' end comprising 2-10 bases or LNA bases. The Rolony Primer-circle hybridization step is carried out at low salt concentrations (< 20 mM monovalent salt) at temperatures between 70°C to 37°C and at low primer concentrations (0.002 µM). The low salt, high specificity hybridization mix is then combined with a high salt enzyme mix, leading to an immediate block of the 3' end of the primer.

### Step e

In this step, the primed circular target DNA molecules are multiplied into DNA concatemers by Rolling Circle Amplification (RCA) reaction.

Preferably, the primed circular target DNA molecules are replicated by a polymerase capable of rolling circle amplification into a plurality of DNA concatemers forming a DNA nanoball or rolony. For this purpose, a primer oligonucleotide is used to prime the binding of the replicating enzyme by hybridizing to a portion of the circular template. In the current approach, a panel of oligonucleotides complementary to only specific regions of the genome (targeted region of the fragmented DNA part of the circular template) are used to prime selective and specific RCA reaction , which would generate a subset of amplified material corresponding a predefine DNA panel of predefine target regions.

Optionally, the DNA concatemers can be localized on a positively charged surface like glass, plastic equivalent and containing polyamines such as silicon dioxide, titanium, hexamethyldisilazane or others. This enables a special detection of the DNA concatemer and in the end or the target DNA. The DNA concatemers may interact to the surface via electrostatic charges or via NHS ester-activated crosslinkers.

### Further embodiments of the method

In an optional further process step, the sequences of nucleotides of the DNA concatemers is determined. For example, the sequences of nucleotides of the multiplied primed circular templates can be determined by sequencing by synthesis using fluorescently labeled oligonucleotides. The labeled oligonucleotides are designed to be complementary to the DNA target of interest that have been selectively and specifically amplified into concatemers by RCA. By hybridizing to its complementary sequence, it will allow for detection of the rolonies via fluorescence signal emission after excitation with the wavelength corresponding to the actual fluorophore label that is tethered to the oligonucleotide.

In addition or in alternative to sequencing, the DNA concatemers selectively amplified by RCA may be detected using fluorescently labeled oligonucleotides. These oligonucleotides are complementary to the specific regions of the genome (targeted region of the fragmented DNA that are part of the circular template) and they share the same sequence as the oligonucleotides that have been used to selectively and specifically amplified the circular template into concatemers by RCA. By hybridizing to its complementary sequence, these labeled oligonucleotides will allow for detection of the rolonies via fluorescence signal emission after excitation with the wavelength corresponding to the actual fluorophore label that is tethered to the oligonucleotide.

### USE

The method is especially useful for samples comprises pathogen DNA molecules or DNA molecules originating from human cancer patients. Pathogens may be viruses or bacteria containing DNA molecules.

### EXAMPLE

The current approach was used to selective amplify a specific region of the genes CD3, CD4, CD8 and GAPDH. Male human genomic DNA (Promega, Madison) was sheared in a sonicator to approximate 150 bp (Covaris, Woburn) and further sized using targeted DNA Size Selection (Pippin Prep, Sage, Beverly, MA).

40 ng of the size selected DNA was used to produce and amplify a shot gun library. To that end, we used a commercial library preparation kit (Qiagen, GeneRead^{™} DNA Library Q) to carry out end repair, barcode - and adaptor ligation and amplification. The efficiency of the process was monitored by confirming the adaptor ligation caused size increase of the DNA using an Agilent Bioanalyzer 2100. 80 ng of the amplified, barcoded and adapter modified double stranded DNA library was incubated with 71 of the R-Bridge oligo at 20 □M. 5'phospho-GTA AAT TGA AGT TGT AAA ACG ACG GCC AGT ddC in 50 □1 in ddH2O which is complementary to each ends of the adapted fragments to be circularized. DNA was separated into single strands at 95 °C (3 min) in a PCR machine; a cold shock (4 °C for 3 min) was applied to hybridize the R-Bridge to the ends of the inverse complimentary ends of the library, hence forming a circle with one half of the ssDNA. To stabilize the circle, the DNA was ligated by addition of 800 units of T4 DNA Ligase (NEB), the corresponding T4 DNA Ligase Buffer with ddH2O to 100 □1 and incubation at 25 °C for 30 min. To remove superfluous DNA likely to prime RCA production in the subsequent steps, the reaction was further treated with Exo I (200 units), Exo III (100 units) and Lambda Exonuclease (10 units) for 60 min. Single stranded circles were purified using the QIAquick PCR kit (Qiagen) and eluted in ddH2O. The purified ssDNA circles were provided gene specific primers for GAPDH, CD3, CD4 and CD8. End concentration of monovalent salts was 15 mM. The primers used were at 0.002 □M end-concentration, for CD4, TGG CTA ATT GAG TAC CCA CCA CTA GCC A, CD8, GAG GGT CCC TCC AGC TAC TGC TC, CD3, CTC AGC TGC TGA GTG AAA GAG GAT ATA TTT ATT GGC TGA G and GAPDH, ACA AGA GGC CTA GGG AAC TCC ATC TTG T.

Hybridization was carried out in a PCR machine with a denaturation step (70°C, 5 min, 37°C, 15 min.). The hybridization mixture was kept at 37°C and 50 □1 of a 2x RCA enzyme mixture was brought to 37°C and added to the 50 1 hybridization mix. The 2xRCA enzyme mix consisted of 600 mM Trehalose, 2 mM Aminoallyl-UTP (Sigma), 20 mM dNTP, 20 ul of 10xPhi29 DNA Pol Buffer and 100U of Phi29 (Enzymatics). The RCA reaction was allowed to proceed for 4.5 hours at 37°C. The reaction was stopped by addition of EDTA to 0.1 M and the rolonies were sequenced. Fq files were generated using proprietary software and the number of specific loci vs. reads aligning with other human genes (i.e. misprimed loci) was determined using CLC (Aarhus, Denmark).

The targeted loci were identified during sequencing proving that this method can be successfully used for specific amplifying target regions of interest within a sample comprising non-target regions.

## Claims

1. A method for selective amplifying target double-stranded DNA molecules from a sample comprising target and non-target DNA molecules **characterized by** subsequently
a. providing target and non-target DNA molecules at the 3' and 5' ends with barcoding regions A1 and A2 each comprising 5 to 50 nucleotides thereby obtaining barcoded DNA molecules
b. providing barcoded DNA molecules with oligonucleotide guides comprising 5 to 50 nucleotides capable of binding to the regions A1 and A2 of the same barcoded DNA molecule and circularizing the barcoded DNA molecules by ligation with a DNA ligase using the guide oligonucleotides thereby obtaining circular templates
c. providing the guide oligonucleotides of the circular templates with a blocking molecule
d. providing the circular templates with a primer oligonucleotide comprising 5 to 50 nucleotides capable of binding at least a part of selected target DNA molecules thereby obtaining primed circular target DNA molecules.
e. multiplying the primed circular target DNA molecules into DNA concatemers by (rolling circle amplification) RCA reaction.

2. Method according to claim 1 **characterized in that** the sequences of nucleotides of the DNA concatemers is determined.

3. Method according to claim 2 **characterized in that** the sequences of nucleotides of the multiplied primed circular templates is determined by sequencing by synthesis using fluorescently labeled oligonucleotides.

4. Method according to any of the claims 1 to 3 **characterized in that** the DNA concatemers are detected using flurorescently labeled oligonucleotide complementary to the targeted fragment.

5. Method according to any of the claims 1 to 4 **characterized in that** the sample comprising target and non-target oligonucleotides is obtained by segmentation/fragmentation of a double stranded DNA nucleic acid library

6. Method according to any of the claims 1 to 5 **characterized in that** the sample comprising target and non-target non-target DNA molecules is obtained by denaturation of a double stranded DNA nucleic acid library into a mixture of sense and anti-sense DNA single strands comprising target and non-target non-target DNA molecules.

7. Method according to any of the claims 1 to 6 **characterized in that** A1 comprises further a random DNA sequence region.

8. Method according to any of the claims 1 to 7 **characterized in that** in step c, the guide oligonucleotides of the circular templates are provided at the 3'end with a blocking molecule wherein the blocking molecule is a dideoxynucleotides capable of blocking the guide oligonucleotides as primer for the RCA reaction in step e).

9. Method according to any of the claims 1 to 8 **characterized in that** the DNA concatemers are localized on a positively charged surface

10. Method according to claim 9 **characterized in that** the DNA concatemers interact to the surface via electrostatic charges or via NHS ester-activated crosslinkers.

11. Method according to any of the claims 1 to 10, **characterized in that** the sample comprises pathogen DNA molecules

12. Method according to claim 11, **characterized in that** the pathogens are viruses, bacterias containing DNA molecules.
